# EUROPEAN PATENT APPLICATION

(11) **EP 4 582 027 A1**
(43) Date of publication of application: **09.07.2025**
(21) Application number: 24150724.3
(22) Date of filing: 08.01.2024
(51) Int. Cl.: A61B 6/10, A61B 6/00

(54) **ASSESSMENT OF RADIATION PROTECTIVE EQUIPMENT PLACEMENT**

(71) Applicant: Koninklijke Philips N.V., 5656 AG Eindhoven (NL)
(72) Inventor: GOOßEN, Andre, Eindhoven (NL); KROENKE-HILLE, Sven, Eindhoven (NL); BRUECK, Heiner Matthias, 5656AG Eindhoven (NL); VON BERG, Jens, Eindhoven (NL); HARDER, Tim, Eindhoven (NL); YOUNG, Stewart Matthew, Eindhoven (NL); BYSTROV, Daniel, Eindhoven (NL); DÖPKE, Max, Eindhoven (NL)
(74) Representative: Philips Intellectual Property & Standards

(57) **Abstract**

The invention is related to a system (100) and a method to assess effectiveness of radiation protection equipment in X-ray imaging exams. The system according to the invention comprises an optical sensor (132) configured to acquire an optical image and a processor (140) in communication with the optical sensor and configured to determine a desired X-ray image responsive to a user input or based on a subject position and/or pose with respect to the X-ray imaging system in the optical image; and determine an effectiveness of a placement of the radiation protection equipment with respect to the subject and/or the X-ray imaging system for the desired X-ray image.

## Description

### FIELD OF THE INVENTION

The invention relates to X-ray imaging and radiation protective equipment.

### BACKGROUND OF THE INVENTION

In daily routine and especially in stressful situations radiation protection might be carried out in inappropriate ways, e.g., forgetting to put on the protective devices or misplacing them. Silva et al. 2010 "The Use and Relevance of Gonad Protection Shields in Children during Hips Radiography." on pediatric exams found that 40% of patients are completely unprotected and in 80% of the cases where a gonad protector was used it was incorrectly placed. This drastically reduces the effects of protective devices and in the worst case can even lead to repeated exams if the incorrectly placed device covers important anatomical structures.

### SUMMARY OF THE INVENTION

It is, inter alia, an object of the invention to assist in radiation protection of subjects subjected to radiation during X-ray imaging.

The invention is defined by the independent claims. Advantageous embodiments are defined in the dependent claims.

In an aspect of the invention there is disclosed a system to assess effectiveness of radiation protection equipment in X-ray imaging exams, the system comprising:
- an optical sensor configured to:
   - acquire an optical image comprising at least:
      - a subject,
      - the subject's position with respect to an X-ray imaging system,
      - a radiation protection equipment, and
      - the radiation protection equipment's position with respect to the subject and/or the X-ray imaging system; and
- a processor in communication with the optical sensor, the processor configured to:
   - determine a desired X-ray image responsive to a user input or based on a subject position and/or pose with respect to the X-ray imaging system; and
   - determine an effectiveness of a placement of the radiation protection equipment with respect to the subject and/or the X-ray imaging system for the desired X-ray image.

In this manner, an effectiveness of a radiation protection equipment is detected which may be communicated to an operator of an X-ray imaging system. For example, if a too low effectiveness is determined, a warning may be issued to re-position the radiation protection equipment. Additionally or alternatively, the X-ray imaging system may only allow a radiation image to be acquired once a satisfactory effectiveness of the radiation protection equipment is determined.

In an example the effectiveness of the placement of the radiation protection equipment is based on a predetermined ideal placement of the radiation protection equipment with respect to the subject and/or the X-ray imaging system for the desired X-ray image.

In an example the processor is further configured to:
- determine a placement suggestion for the radiation protection equipment with respect to the subject and/or the X-ray imaging system for the desired X-ray image, wherein the placement suggestion has a higher effectiveness than the initially determined effectiveness of the placement.

In an example the processor is further configured to:
- determine a placement guidance to reach the placement suggestion for the radiation protection equipment with respect to the subject and/or the X-ray imaging system for the desired X-ray image; and
wherein the system further comprises:
- at least one first output device to output the placement guidance to reach the placement suggestion of the radiation protection equipment with respect to the subject and/or the X-ray imaging system for the desired X-ray image, wherein the at least one output device comprises any one or a combination of:
   - at least one visual output device,
   - at least one audible output device, and
   - at least one haptic output device.

In this manner, a radiographer can be assisted in re-positioning for example a gonad or a lead apron.

In an example the output device is mounted on the radiation protection equipment.

In this manner, the transmission of the effectiveness and/or the instructions to improve the effectiveness of the radiation protection equipment may be transmitted to a radiographer quicker and in a more intuitive manner. For example, if correctly placed, a lead apron may immediately display several green LEDs, giving immediate feedback after placement. Alternatively, if incorrectly placed, LEDs of a lead apron may be colored red intuitively and immediately providing feedback to the radiographer that the positioning of the apron is ineffective. Additionally, if said LEDs are positioned on edges of the apron, by turning some LEDs green and others red, the radiographer may instantaneously receive guidance to move the apron into one or another direction.

In an example the processor is further configured to:
- determine an effectiveness of a position and/or pose of the subject with respect to the X-ray imaging system for the desired X-ray image.

In an example the effectiveness of the position and/or pose of the subject with respect to the X-ray imaging system for the desired X-ray image is based on a predetermined ideal position and/or pose of the subject with respect to the X-ray imaging system for the desired X-ray image.

In an example the processor is further configured to:
- determine a position and/or pose suggestion of the subject with respect to the X-ray imaging system for the desired X-ray image, wherein the position and/or pose suggestion has a higher effectiveness than the initially determined effectiveness of the position and/or pose.

In an example the processor is further configured to:
- determine a positioning guidance to reach the position and/or pose suggestion of the subject with respect to the X-ray imaging system for the desired X-ray image; and
- wherein the system further comprises:
   - at least one second output device to output the positioning guidance to reach the position and/or pose suggestion of the subject with respect to the X-ray imaging system for the desired X-ray image, wherein the at least one output device comprises any one or a combination of:
      - at least one visual output device,
      - at least one audible output device, and
      - at least one haptic output device.

In an example the at least one visual output device comprises any one or a combination of:
- at least one display,
- at least one light, and
- at least one projector.

In an example the at least one projector is configured to project:
- an outline of the placement guidance to reach the placement suggestion of the radiation protection equipment with respect to the subject and/or the X-ray imaging system for the desired X-ray image; or
- an outline of the positioning guidance to reach the position and/or pose suggestion of the subject with respect to the X-ray imaging system for the desired X-ray image.

In an example the radiation protection equipment further comprises sensors configured to assist in determining the effectiveness of the placement of the radiation protection equipment with respect to the subject and/or the X-ray imaging system for the desired X-ray image.

In an aspect of the invention there is disclosed a radiation protection equipment comprising at least one output device as disclosed herein and/or at least one sensor as disclosed herein.

In an aspect of the invention there is disclosed a computer implemented method, the method comprising:
- receiving an optical image comprising a subject, the subject's position with respect to an X-ray imaging system, a radiation protection equipment, and the radiation protection equipment's position with respect to the subject and/or the X-ray imaging system;
- determining a desired X-ray image responsive to a user input or based on a subject position and/or pose with respect to the X-ray imaging system; and
- determining an effectiveness of a placement of the radiation protection equipment with respect to the subject and/or the X-ray imaging system for the desired X-ray image.

In an aspect of the invention there is disclosed a computer program product or non-transitory medium comprising instructions that when executed by a processor, cause the processor to perform all of the steps of the method according to any of the herein described methods.

These and other aspects of the invention will be apparent from and elucidated with reference to the embodiments described hereinafter.

### BRIEF DESCRIPTION OF THE DRAWINGS

Fig. 1 shows a schematic of a system and subject positioned for an X-ray imaging exam in accordance with an embodiment of the invention.
Fig. 2 shows an X-ray image comprising a gonad protector.
Fig. 3 shows a schematic of a radiation protection equipment in accordance with an embodiment of the invention.
Fig. 4 shows a method in accordance with an embodiment of the invention.
Fig. 5 shows a processor circuit in accordance with an embodiment of the invention.

### DETAILED DESCRIPTION OF EMBODIMENTS

The invention relates to assessing effectiveness of radiation protection equipment in X-ray imaging. The inventors have recognized that radiation protection equipment is frequently not positioned correctly, which leads to unnecessary radiation exposure of subjects during X-ray imaging exams and in some cases to re-takes, since the radiation protection equipment may obscure features of interest. Assessment of the placement of radiation protection equipment in X-ray imaging can thus be of significant help to a person placing the radiation protection equipment. The system according to the invention does thus determine an effectiveness of a placement of the radiation protection equipment and may communicate this effectiveness to for example the radiographer.

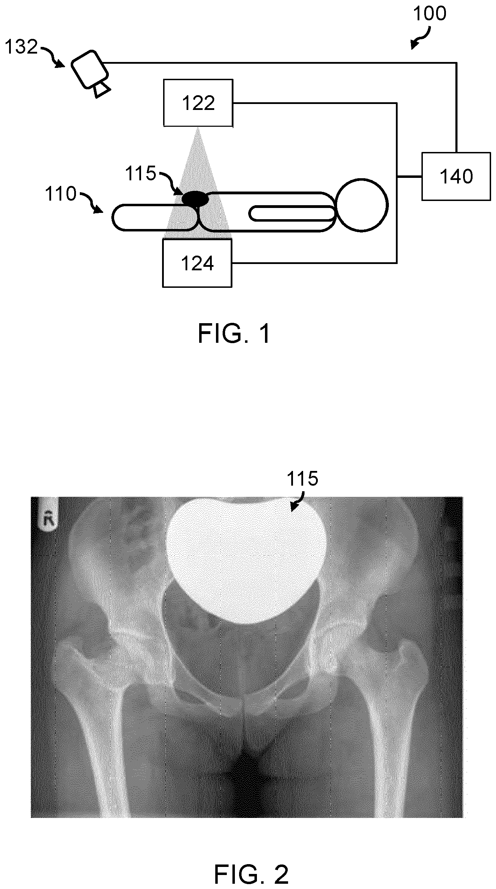

According to the invention, Fig. 1 discloses a system 100 that may be used to assess effectiveness of radiation protection equipment in X-ray imaging exams, the system comprising:
- an optical sensor 132 configured to:
   - acquire an optical image comprising at least:
      - a subject 110 with respect to an X-ray imaging system, and
      - a radiation protection equipment 115 with respect to the subject and/or the X-ray imaging system; and
- a processor 140 in communication with the optical sensor, the processor configured to:
   - determine an X-ray imaging exam responsive to a user input or based on a subject position and/or pose with respect to the X-ray imaging system; and
   - determine an effectiveness of a placement of the radiation protection equipment with respect to the subject and/or the X-ray imaging system for the determined X-ray imaging exam.

In this manner, if it is determined that the placement of the radiation protection equipment is ineffective, a warning can be issued to a radiographer.

The optical sensor 132 may be any suitable optical sensor including but not limited to imaging sensors found in digital or analogue cameras and in thermal cameras. The optical image may respectively be, for example, a natural image like a photograph or a thermal image. Preferably the optical image shows a subject with respect to the X-ray imaging sensor (source and/or detector). For example, the optical image may display a subject lying on an examination table of the X-ray imaging system. In another example, the optical image may display the subject standing against the detector or source of the X-ray imaging system. The optical image is further configured to comprise a radiation protection equipment 115 like a lead apron. An X-ray imaging exam according to this specification comprises at least a desired X-ray image. For example, a determined X-ray imaging exam of a lung may comprise one or more desired lung X-ray images, potentially from different angles.

The radiation protection equipment 115 may be any suitable radiation protection equipment, but most commonly a lead apron used to shield particular regions of the subject from radiation. Alternative radiation protection equipment known to the skilled person are lead glasses, for protecting the eyes of personnel or subject, lead gloves for protecting the hands of personnel or subject, thyroid shields for protecting the thyroid glands and lead caps for protecting the head. While lead is the most commonly used material, radiation protection equipment comprising bismuth-based materials, tungsten and tungsten alloys, composite materials, ceramic based materials, and even biodegradable materials are known.

The X-ray imaging system may be any suitable X-ray imaging system comprising for example an X-ray source 122 from which radiation may be emitted and a detector 124 to detect radiation. The detector may also be a separate detector not part of the X-ray imaging system. Commonly, the subject is positioned between the source and detector for taking an X-ray image. While in Fig. 1, the source and detector are shown vertical with respect to each other, a horizontal alignment, or any other diagonal alignment is also envisaged by the current disclosure. A representative X-ray image taken of a pelvis-examination including a radiation protection equipment 115 is shown in Fig. 2. The position of the subject with respect to the X-ray imaging system may thus refer to the position of the subject with respect to the X-ray radiation source 122.

The processor 140 of system 100 may be any suitable processor in communication, wired or wireless, with the optical sensor 132. The processor may for instance be a part of the X-ray imaging system or may be a separate processor located within proximity (e.g., same room) or remotely from the X-ray imaging system. The processing performed by the processor 140 may thus be for example performed on the cloud.

The processor 140 may be configured to determine an X-ray imaging exam responsive to a user input. For example, a user may input a lung X-ray examination via an input interface of the X-ray imaging system, which input is conveyed to the processor 140.

The processor 140 may additionally or alternatively be configured to determine an X-ray imaging exam based on a subject position and/or pose with respect to the X-ray imaging system. For example, based on a positioning of the subject determined from the optical image, and the position and configuration of the X-ray source and detector, the processor may determine that configuration is representative of a particular X-ray imaging exam. For example, when the subject is lying or sitting on an examination table and the X-ray source is positioned over the knee, the processor may determine the configuration corresponds to that for taking a knee X-ray image. Similarly, when the X-ray source and detector are positioned in a position with respect to the subject that is known to be relevant for a chest X-ray imaging exam, the processor may determine that a chest X-ray imaging exam is to be performed.

The determination of processor 140 based on a subject position and/or pose with respect to the X-ray imaging system may take place according to pre-defined rules as described above, or it may comprise a trained machine learning model. For instance, a neural network may be trained with input images similar to the optical images described above and a ground truth representative of the respective X-ray imaging exam. The optical image may comprise a view of the source and/or detector. Additionally or alternatively, the location of the source and/or detector may be transmitted to the processor separately to be included in the determination of the position and/or pose of the subject with respect to the X-ray imaging system. A suitable algorithm that may be used to determine a subject position and/or pose with respect to the X-ray imaging system is the regression model from Sénégas et al. 2018 "Evaluation of Collimation Prediction Based on Depth Images and Automated Landmark Detection for Routine Clinical Chest X-Ray Exams".

According to the invention, processor 140 is further configured to determine an effectiveness of a placement of the radiation protection equipment with respect to the subject and/or the X-ray imaging system for the determined X-ray imaging exam. For example, in the previous step of determining the X-ray imaging exam type, the processor may have determined a pelvis-exam is to be performed. In this step, the processor 140 may then further identify a radiation protection equipment covering the reproductive organs of a subject and may generate an effectiveness measure of the placement.

Identifying the radiation protection equipment in the optical image may occur via any known means. For example, using segmentation algorithms such as model based segmentation, convolutional neural network segmentation algorithms, etc. In a preferred embodiment a convolutional neural network with a You Only Look Once (YOLO) architecture is used for detection of the radiation protection equipment.

According to an example the effectiveness of the placement of the radiation protection equipment is based on a predetermined ideal placement of the radiation protection equipment with respect to the subject and/or the X-ray imaging system for the determined X-ray imaging exam. For example, for said determined pelvis-exam, an ideal placement of a lead apron, i.e., gonad protection, may be known which can be compared to the placement identified in the optical image. Based on any such comparison, an effectiveness of the current placement is derived. The measure of effectiveness may thus for example be determined by an overlap of the current placement and the ideal placement, wherein total overlap would yield 100% effectiveness, partial overlap would yield an effectiveness between 0 and 100% and no overlap at all would yield an effectiveness of 0%. Accordingly, it may be determined that an effectiveness above 95% is considered good yielding for example a green indicator, an effectiveness between 85 and 95% may be considered sufficient yielding for example a yellow indicator, and an effectiveness below 85% may be considered insufficient yielding for example a red indicator, wherein the indicators may be displayed on a display, or a light as described in more detail below.

According to an example, the processor is further configured to determine a new placement, i.e., a placement suggestion, of the radiation protection equipment with respect to the subject and/or the X-ray imaging system for the determined X-ray imaging exam, wherein the new placement has a higher effectiveness than the effectiveness of the placement.

According to an example, the processor is further configured to determine an instruction, i.e., a placement guidance, to reach the new placement of the radiation protection equipment with respect to the subject and/or the X-ray imaging system for the determined X-ray imaging exam; and
wherein the system further comprises at least one first output device to output the instruction to reach the new placement of the radiation protection equipment with respect to the subject and/or the X-ray imaging system for the determined X-ray imaging exam, wherein the at least one output device comprises any one or a combination of:
- at least one visual output device,
- at least one audible output device, and
- at least one haptic output device.

According to an example, the at least one visual output device comprises any one or a combination of:
- at least one display,
- at least one light, and
- at least one projector.

The at least one first output device may thus comprise a display that displays an image, or a model of the subject for privacy reasons, with a predetermined ideal and/or a current placement of the radiation protection equipment. Said display may additionally display other optical guidance components like arrows or color-coded lights (e.g., red for not well placed and green for well placed) suggesting movements on the radiation protection equipment. Said display may additionally or alternatively also display a sequence (e.g., an animation with a moving outline from a current placement to an ideal placement, for example, similar to a GIF (Graphics Interchange Format)) suggesting motion of the radiation protection equipment from the current placement to an ideal placement. It is understood that a light throughout this specification comprises a light source.

The at least one first output device may thus comprise a light that changes color respective to the effectiveness of the radiation protection equipment placement.

The at least one first output device may thus comprise a projector which projects instructions onto the subject and/or radiation protection equipment. For example, the projector may display an ideal placement of the radiation protection equipment, thereby guiding the placement. Additionally or alternatively, the projector may also display guiding components including arrows, or image sequences, wherein the image sequence comprises a suggested movement of the radiation protection equipment to reach the ideal placement when starting at the current placement.

According to an example, a projector is configured to project an outline of the instruction to reach the new placement of the radiation protection equipment with respect to the subject and/or the X-ray imaging system for the determined X-ray imaging exam.

The at least one first output device may thus comprise a speaker through which voice commands may be conveyed to the person responsible for placing the radiation protection equipment.

The at least one first output device may comprise a haptic device. For example, a device worn by the person placing the radiation protection equipment may thus vibrate to indicate effective and/or ineffective placement of the radiation protection equipment.

According to an example the output device is mounted on the radiation protection equipment. In other words, the radiation protection equipment may comprise signaling means to convey information to the person responsible for the radiation protection equipment placement.

The at least one first output device may thus comprise one or multiple displays mounted on the radiation protection equipment to display information. For example, to display arrows on how to move a particular edge of said radiation protection equipment. For example, to display colors to indicate effective (e.g., represented with green) and/or ineffective (e.g., represented with red) placement, or mediocre placement (e.g., represented with yellow). It should be understood that lights and arrows may also be displayed by light sources such as LEDs.

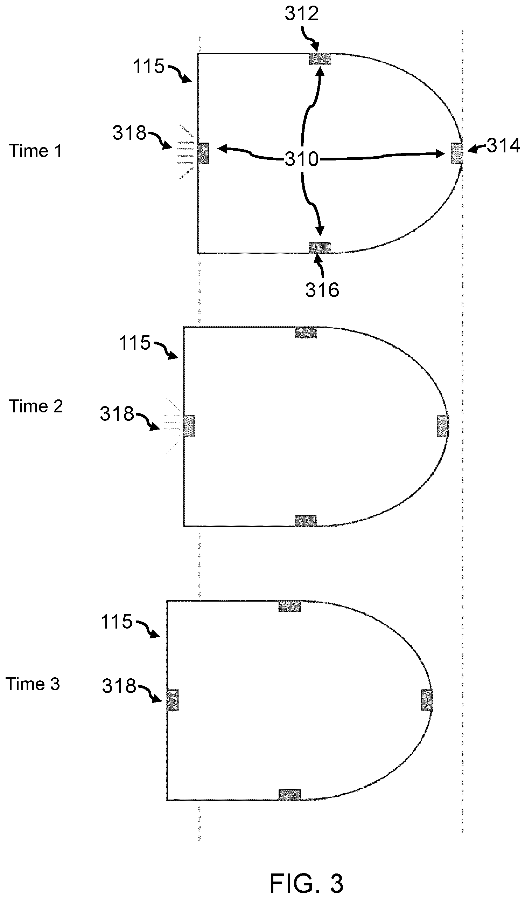

Fig. 3 shows an exemplary apron as radiation protection equipment with LEDs 310 that signal placement effectiveness. The apron according to Fig. 3 is moved from a Time 1 to a Time 3 to improve the effectiveness of the placement of the apron.

In the top of Fig. 3, at Time 1, the apron is not effectively placed on the horizontal axes, while it is effectively placed on the vertical axes. Therefore, the LEDs 312 and 316 are displayed in green, while LED 314 is shown in yellow and LED 318 in red. Thereby an instruction is generated to move the apron to the left. To further emphasize this instruction, LED 318 may be blinking.

In the middle of Fig. 3, at Time 2, the horizontal placement of the apron has improved, such that the color of LED 318 is switched to yellow. Nonetheless, LED 318 may remain blinking to keep the instruction to move into that direction. The operator is thus informed that he/she is moving the apron in the right direction, and that now the movement speed may be reduced as the current position is getting close to the ideal placement.

In the bottom of Fig. 3, at Time 3, the placement of the apron may have been determined to be ideal. Therefore all LEDs are displayed in green.

It is obvious to a skilled person to utilize these teachings to also implement other signaling means including but not limited to changing light intensity, blinking, light color and displays instead of LEDs, without departing from the inventiveness of the smart radiation protection equipment.

The at least one first output device may thus comprise a projector which projects instructions on edges of the radiation protection equipment. For example, projectors on the edges of the radiation protection equipment may display outlines of the radiation protection equipment, arrows or color-coded lights, onto the subject body or examination table to guide the placement. It should be understood that such a projecting function may also be achieved by light sources such as LEDs
The at least one first output device may thus comprise a speaker through which guidance may be conveyed to the person responsible for placing the radiation protection equipment. Such guidance may be similar to for example a car's park distance beeping. For example, various speakers on multiple edges may beep if not positioned correctly. Alternatively, the various speakers may beep when positioned correctly. It is to be understood that the various speakers may function independently, such that individual placement guidance on each edge of the radiation protection equipment may be given.

A similar effect as the park-distance beeping of the radiation protection equipment may also be achieved via a plurality of haptic devices on the edges of the radiation protection equipment, wherein edges may vibrate upon effective or ineffective placement.

Radiation protection equipment with an output device as described herein may be converted called smart radiation protection equipment. This smart radiation protection equipment may be in communication with the processor 140, enabling placement guidance to be given in an intuitive manner, without the person responsible for placing the radiation protection equipment requiring to look up, for example to a display, during placement. Thereby potentially reducing the workload of, for example radiographers.

According to an example the processor is further configured to determine an effectiveness of a position and/or pose of the subject with respect to the X-ray imaging system for the determined X-ray imaging exam.

According to an example the effectiveness of the position and/or pose of the subject with respect to the X-ray imaging system for the determined X-ray imaging exam is based on an ideal position and/or pose of the subject with respect to the X-ray imaging system for the determined X-ray imaging exam.

According to an example, the processor is further configured to determine a new position and/or pose, i.e., a position and/or pose suggestion, of the subject with respect to the X-ray imaging system for the determined X-ray imaging exam, wherein the new position and/or pose has a higher effectiveness than the effectiveness of the position and/or pose.

For example, in determining a positioning and/or pose effectiveness or guidance, processor 140 may implement a rule-based approach, a statistical approach or a machine learning model to determine the effectiveness and/or any guidance to improve said effectiveness. For example, a machine learning model may be trained with video feed of subject positioning, including pose, for particular X-ray exams.

According to an example the processor is further configured to:
- determine an instruction, i.e., a positioning guidance to reach the new position and/or pose of the subject with respect to the X-ray imaging system for the determined X-ray imaging exam; and
- wherein the system further comprises:
   - at least one second output device to output the instructions to reach the new position and/or pose of the subject with respect to the X-ray imaging system for the determined X-ray imaging exam, wherein the at least one output device comprises any one or a combination of:
      - at least one visual output device,
      - at least one audible output device, and
      - at least one haptic output device.

The first and second output devices may be the same or different output devices.

According to an example the at least one visual output device comprises any one or a combination of:
- at least one display,
- at least one light, and
- at least one projector.

The effectiveness of the subject position and/or pose and/or guidance to improve the effectiveness of the subject position and/or pose, may thus be conveyed to the responsible personnel or the subject via a display that displays an image, or a model of the subject for privacy reasons, with an ideal and/or a current position and/or pose of the subject. Said display may additionally display other optical guidance components like arrows or color-coded lights (e.g., red for not well placed and green for well placed) suggesting movements of the subject. Said display may additionally or alternatively also display a sequence (e.g., a GIF showing a moving outline of a subject from a current to an ideal position and/or pose) suggesting motion of the subject from the current position and/or pose to an ideal position and/or pose.

The effectiveness of the subject position and/or pose and/or guidance to improve the effectiveness of the subject position and/or pose, may thus be conveyed to the responsible personnel or the subject via a light that changes color respective to the effectiveness of the subject position and/or pose.

According to an example, the at least one projector is configured to project an outline of the instruction to reach the new position and/or pose of the subject with respect to the X-ray imaging system for the determined X-ray imaging exam.

The effectiveness of the subject position and/or pose and/or guidance to improve the effectiveness of the subject position and/or pose, may thus be conveyed to the responsible personnel or the subject via a projector which projects instructions onto the subject and/or examination table. For example, the projector may display an outline of an ideal subject position and/or pose on the examination table, thereby guiding the placement. Additionally or alternatively, the projector may also display guiding components including arrows, or image sequences, wherein the image sequence comprises a suggested movement of the subject to reach the ideal position and/or pose when starting at the current position and/or pose.

The effectiveness of the subject position and/or pose and/or guidance to improve the effectiveness of the subject position and/or pose, may thus be conveyed to the responsible personnel or the subject via a speaker through which voice commands may be conveyed.

The effectiveness of the subject position and/or pose and/or guidance to improve the effectiveness of the subject position and/or pose, may thus be conveyed to the responsible personnel or the subject via a haptic device. For example, a device worn by the person positioning the subject or by the subject themselves. Additionally or alternatively, for example the examination table may comprise a haptic device configured to vibrate either if the subject position and/or placement is determined to be ineffective, or to give a single vibration when an effective subject position and/or pose is reached.

It should be understood that the output devices responsible for conveying the effectiveness of the subject position and/or pose and/or guidance to improve the effectiveness of the subject position and/or pose may also be implemented on the radiation protection equipment. In such cases, a display, light source, projector, speaker, or haptic device mounted on the radiation protection equipment may display the herein described guidance.

According to an example, the radiation protection equipment further comprises sensors configured to assist in determining the effectiveness of the placement of the radiation protection equipment with respect to the subject and/or the X-ray imaging system for the determined X-ray imaging exam. For example, the effectiveness of the placement of the radiation protection equipment and/or the guidance to improve said effectiveness may be further determined by processor 140 with the assistance of sensors on the radiation protection equipment. For example, the radiation protection equipment may comprise inertial measurement units, accelerometers and/or optical cameras that can be used in determining the effectiveness of the placement of the radiation protection equipment.

According to an embodiment, there is provided a radiation protection equipment comprising at least one output device and/or at least one sensor as disclosed herein. For example, there may be provided a lead apron comprising LEDs in the edges to indicate effective or ineffective placement.

According to an embodiment there is disclosed a method 400 comprising:
- receiving 420 an optical image comprising: a subject, the subject's position with respect to an X-ray imaging system, a radiation protection equipment, and the radiation protection equipment's position with respect to the subject and/or the X-ray imaging system;
- determining 440 an X-ray imaging exam, e.g., a desired X-ray image, responsive to a user input or based on a subject position and/or pose with respect to the X-ray imaging system; and
- determining 460 an effectiveness of a placement of the radiation protection equipment with respect to the subject and/or the X-ray imaging system for the determined X-ray imaging exam; and
- an optional step of determining 480 an instruction to reach the new placement of the radiation protection equipment with respect to the subject and/or the X-ray imaging system for the determined X-ray imaging exam.

According to an embodiment there is provided a computer program product or a non-transitory medium comprising instructions that when executed by a processor cause the processor to perform any of the herein described methods.

According to an embodiment, there is provided a processor to perform any of the herein described methods or steps. This processor may be a stand-alone processor or part of an X-ray imaging system as described with reference to Fig. 1.

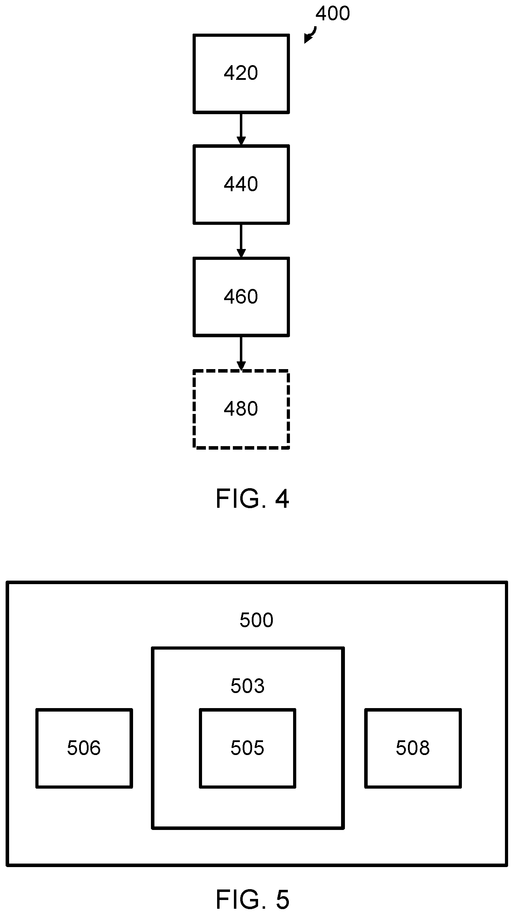

Fig. 5 is a schematic diagram of a processor circuit 500, according to aspects of the present disclosure. As shown, the processor circuit 500 may include a processor 506, a memory 503, and a communication module 508. These elements may be in direct or indirect communication with each other, for example via one or more buses.

The processor 506 as envisaged by the present disclosure may include a central processing unit (CPU), a graphical processing unit (GPU), a digital signal processor (DSP), an application specific integrated circuit (ASIC), a controller, a field programmable gate array (FPGA) device, another hardware device, a firmware device, or any combination thereof configured to perform the operations described herein. The processor 506 may also be implemented as a combination of computing devices, e.g., a combination of a DSP and a microprocessor, a plurality of microprocessors, one or more microprocessors in conjunction with a DSP core, or any other such configuration. The processor 506 may also implement various deep learning networks, which may include a hardware or a software implementation. The processor 506 may additionally include a preprocessor in either hardware or software implementation.

The memory 503 as envisaged by the present disclosure may be any suitable storage device, such as a cache memory (e.g., a cache memory of the processor 506), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. The memory may be distributed among multiple memory devices and/or located remotely with respect to the processor circuit. In an embodiment, the memory 503 may store instructions 505. The instructions 505 may include instructions that, when executed by a processor 506, cause the processor 506 to perform the operations described herein.

Instructions 505 may also be referred to as code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements. Instructions 505 may be in the form of an executable computer program or script. For example, routines, subroutines and/or functions may be defined in a programming language including but not limited to Python, C, C++, C#, Pascal, BASIC, API calls, HTML, XHTML, XML, ASP scripts, JavaScript, FORTRAN, COBOL, Perl, Java, ADA, .NET, and the like. Instructions may also include instructions for machine learning and/or deep learning.

The communication module 608 can include any electronic circuitry and/or logic circuitry to facilitate direct or indirect communication of data between the processor circuit 500, and for example an external display (not shown) and/or an imaging device or system such as an X-ray imaging system. In that regard, the communication module 608 can be an input/output (I/O) device. The communication may be performed via any suitable means. For example, the communication means may be a wired link, such as a universal serial bus (USB) link or an Ethernet link. Alternatively, the communication means may be a wireless link, such as an ultra-wideband (UWB) link, an Institute of Electrical and Electronics Engineers (IEEE) 802.11 wireless network link, or a Bluetooth link.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention may be implemented by means of hardware comprising several distinct elements, and/or by means of a suitably programmed processor. In the device claim enumerating several means, several of these means may be embodied by one and the same item of hardware. Measures recited in mutually different dependent claims may advantageously be used in combination.

## Claims

1. A system (100) to assess effectiveness of radiation protection equipment in X-ray imaging exams, the system comprising:
- an optical sensor (132) configured to:
- acquire an optical image comprising at least:
- a subject (110);
- the subject's position with respect to an X-ray imaging system, and
- a radiation protection equipment (115); and
- the radiation protection equipment's position with respect to the subject and/or the X-ray imaging system; and
- a processor (140) in communication with the optical sensor, the processor configured to:
- determine a desired X-ray image responsive to a user input or based on a subject position and/or pose with respect to the X-ray imaging system; and
- determine an effectiveness of a placement of the radiation protection equipment with respect to the subject and/or the X-ray imaging system for the desired X-ray image.

2. The system of claim 1, wherein the effectiveness of the placement of the radiation protection equipment is based on a predetermined ideal placement of the radiation protection equipment with respect to the subject and/or the X-ray imaging system for the desired X-ray image.

3. The system of claim 1 or 2, wherein the processor is further configured to:
- determine a placement suggestion for the radiation protection equipment with respect to the subject and/or the X-ray imaging system for the desired X-ray image, wherein the placement suggestion has a higher effectiveness than the initially determined effectiveness of the placement.

4. The system of any one of claims 1 to 3, wherein the processor is further configured to:
- determine a placement guidance to reach the placement suggestion of the radiation protection equipment with respect to the subject and/or the X-ray imaging system for the desired X-ray image; and
wherein the system further comprises:
- at least one first output device to output the placement guidance to reach the placement suggestion of the radiation protection equipment with respect to the subject and/or the X-ray imaging system for the desired X-ray image, wherein the at least one output device comprises any one or a combination of:
- at least one visual output device,
- at least one audible output device, and
- at least one haptic output device.

5. The system of claim 4, wherein the output device is mounted on the radiation protection equipment.

6. The system of any one of claims 1 to 5, wherein the processor is further configured to:
- determine an effectiveness of a position and/or pose of the subject with respect to the X-ray imaging system for the desired X-ray image.

7. The system of claim 6, wherein the effectiveness of the position and/or pose of the subject with respect to the X-ray imaging system for the desired X-ray image is based on a predetermined ideal position and/or pose of the subject with respect to the X-ray imaging system for the desired X-ray image.

8. The system of claim 7, wherein the processor is further configured to:
- determine a position and/or pose suggestion of the subject with respect to the X-ray imaging system for the desired X-ray image, wherein the position and/or pose suggestion has a higher effectiveness than the initially determined effectiveness of the position and/or pose.

9. The system of claim 8, wherein the processor is further configured to:
- determine a positioning guidance to reach the position and/or pose suggestion of the subject with respect to the X-ray imaging system for the desired X-ray image; and
- wherein the system further comprises:
- at least one second output device to output the positioning guidance to reach the position and/or pose suggestion of the subject with respect to the X-ray imaging system for the desired X-ray image, wherein the at least one output device comprises any one or a combination of:
- at least one visual output device,
- at least one audible output device, and
- at least one haptic output device.

10. The system of claim 4 or 9, wherein the at least one visual output device comprises any one or a combination of:
- at least one display,
- at least one light, and
- at least one projector.

11. The system of claim 10, wherein the at least one projector is configured to project:
- an outline of the placement guidance to reach the placement suggestion of the radiation protection equipment with respect to the subject and/or the X-ray imaging system for the desired X-ray image; or
- an outline of the positioning guidance to reach the position and/or pose suggestion of the subject with respect to the X-ray imaging system for the desired X-ray image.

12. The system of any one of claims 1 to 11, wherein the radiation protection equipment further comprises sensors configured to assist in determining the effectiveness of the placement of the radiation protection equipment with respect to the subject and/or the X-ray imaging system for the desired X-ray image.

13. A radiation protection equipment comprising at least one output device according to claim 5 and/or at least one sensor according to claim 12.

14. A computer implemented method (400), the method comprising:
- receiving (420) an optical image comprising: a subject, the subject's position with respect to an X-ray imaging system, a radiation protection equipment, and the radiation protection equipment's position with respect to the subject and/or the X-ray imaging system;
- determining (440) a desired X-ray image responsive to a user input or based on a subject position and/or pose with respect to the X-ray imaging system; and
- determining (460) an effectiveness of a placement of the radiation protection equipment with respect to the subject and/or the X-ray imaging system for the desired X-ray image.

15. A computer program product comprising instructions that when executed by a processor (140), cause the processor to perform all of the steps of the method according to claim 14.
